# FASCICULE DE BREVET EUROPEEN

(11) **EP 1 865 871 B1**
(45) Date de publication et mention de la délivrance du brevet: **28.03.2012**
(21) Numéro de dépôt: 06707777.6
(22) Date de dépôt: 20.01.2006
(51) Int. Cl.: A61B 18/14

(54) **CAPTEUR D'IMAGE DENTAIRE INTRA-ORAL ET SYSTEME RADIOLOGIQUE UTILISANT CE CAPTEUR**
INTRAORALER DENTALER BILDGEBENDER SENSOR UND DIESEN UMFASSENDES RADIOLOGISCHES SYSTEM
INTRAORAL DENTAL IMAGING SENSOR AND RADIOLOGICAL SYSTEM COMPRISING SAME

(30) Priorité: 01.04.2005 FR 0503182
(43) Date de publication de la demande: 19.12.2007
(73) Titulaire: E2V Semiconductors, 38521 Saint Egreve (FR)
(72) Inventeur: AYRAUD, Michel, F-38340 Voreppe (FR)
(74) Mandataire: Guérin, Michel
(86) Numéro de dépôt international: PCT/EP2006/050329
(87) Numéro de publication internationale: WO 2006/103126

(56) Documents cités:
- WO-A-00/29872
- US-A- 3 622 785
- US-A- 5 434 418
- US-A- 6 030 119
- US-A- 6 042 267

## Description

L'invention concerne les capteurs d'image dentaire radiologiques intra-oraux, c'est-à-dire placés dans la bouche d'un patient, une source de rayons X étant placée à l'extérieur de la joue du patient pour émettre des rayons X dans la direction du capteur.

De manière habituelle, pour transmettre les informations d'image issues du capteur placé à l'intérieur de la bouche, on utilise une connexion filaire qui sert également à la commande du capteur et à son alimentation en énergie.

L'inconvénient d'une connexion filaire est qu'elle est fragile (risque d'arrachage), gênante pour le patient si on tire accidentellement sur le fil, encombrante dans l'installation générale. De plus, en environnement médical, les contraintes d'isolation électrique entre le patient et les alimentations électriques environnantes sont élevées et il est peu conforme à ces contraintes de connecter un appareil (micro-ordinateur) alimenté par le réseau de puissance à un module qui est dans la bouche du patient.

On a donc cherché à réaliser des connexions sans fil à la fois pour amener l'énergie et pour passer les informations en provenance du capteur ou vers le capteur. L'alimentation en énergie sans fil (typiquement : par transmission inductive) étant peu commode, on préfère en général utiliser une batterie (rechargeable ou non) dans le capteur placé dans la bouche. Par ailleurs, les informations d'image issues du capteur doivent être émises à débit élevé (de l'ordre de 20 Mégabits par seconde) et c'est pourquoi on s'oriente vers des transmissions par radiofréquence, de préférence dans les bandes de fréquence libres, qui sont en pratique celles qui servent à la communication informatique sans fil en réseau local (fréquences attribuées aux réseaux WLAN : 2,45 GHz par exemple).

Toutefois, la difficulté vient de ce que cette transmission radio risque alors d'être fortement perturbée par la présence d'autres émetteurs radio de plus en plus fréquemment utilisés dans les environnements informatiques ; des périphériques et cartes d'ordinateur de type "WiFi" ou "bluetooth" peuvent en particulier perturber fortement la transmission des données d'image du capteur vers le système d'exploitation de l'image.

On peut pallier cette difficulté en émettant les messages de manière redondante, pour assurer une transmission complète et sûre de toute l'image, mais cela consomme du temps, alors que les informations à transmettre sont déjà en quantité importante (typiquement : plusieurs dizaines de mégabits par image).

On peut également utiliser un émetteur "intelligent" qui examine quelles sont les fréquences non utilisées localement dans l'environnement et qui adapte sa propre fréquence et/ou son propre débit en fonction de cet environnement. Un tel émetteur comprend nécessairement aussi un récepteur. L'électronique complexe de réception, d'analyse et de traitement intelligent qui en résulte rend très difficile la mise en place de l'ensemble dans la bouche du patient. L'encombrement, la consommation de puissance, sont prohibitives. On est alors obligé de diviser le système en un capteur situé à l'intérieur de la bouche, un fil de liaison qui part du capteur et qui sort à l'extérieur de la bouche, un émetteur-récepteur dans une poche du patient, et une liaison radiofréquence intelligente entre - cet émetteur-récepteur extra-oral et le système d'exploitation (micro-ordinateur) qui doit recueillir les images. Une telle installation est complexe.

Le document US-A-3 622 785 décrit un système selon le préambule de la revendication 1.

Pour éviter ces inconvénients, l'invention part de l'observation que bien que le capteur soit situé à l'intérieur de la bouche, il s'avère quand même possible de transmettre l'information d'image au système sans utiliser de fil pour relier le capteur au système d'exploitation et sans utiliser d'émission radiofréquence.

L'invention propose un système de radiologie dentaire par voie intra orale comportant un capteur d'image radiologique apte à être inséré dans la bouche du patient et comportant d'une part une matrice de détection d'image fournissant des signaux électroniques représentant une image radiologique et d'autre part une source de lumière recevant les signaux issus de la matrice et apte à émettre des impulsions lumineuses binaires correspondant à une image numérique à transmettre par voie optique, et un récepteur de lumière placé à distance du patient, apte à détecter une modulation de lumière provoquée par la source de lumière placée dans la bouche, et apte à transmettre un signal correspondant à cette modulation à un dispositif de traitement d'image (notamment pour assurer le stockage de l'image recueillie).

D'une manière inattendue, on propose donc de transmettre l'information par une source lumineuse bien que la source des informations à transmettre soit dans un environnement (l'intérieur de la bouche) qui devrait exclure une transmission par voie lumineuse.

Dans une première variante, qui a l'avantage de la simplicité, la source de lumière n'émet qu'à l'intérieur de la bouche du patient, mais à une longueur d'onde et avec une puissance telle qu'une fraction de la lumière passe à travers les joues ; cette fraction de lumière se superpose à la lumière ambiante sous forme d'une modulation de cette lumière ambiante, de sorte que le récepteur peut détecter la modulation de la lumière donc l'information d'image transmise par l'intermédiaire de cette modulation. De préférence, la source de lumière est une diode laser ou une diode électroluminescente émettant une lumière sensiblement monochromatique et le récepteur est pourvu d'un filtre optique à bande passante étroite centrée autour de la longueur d'onde de la lumière monochromatique. La longueur d'onde utilisée est dans la bande de 700 à 900 nanomètres, de préférence 780 à 880 nanomètres (rouge ou proche-infrarouge), bande dans laquelle la peau des joues laisse passer une fraction notable de la lumière émise.

Une solution très avantageuse du point de vue de la transmission, du fait qu'elle n'est pas affectée par le taux de transmission optique des joues, consiste à placer en regard de la source de lumière (là encore de préférence une diode laser ou électroluminescente à émission sensiblement monochromatique) une première extrémité d'une fibre optique, à l'intérieur de la bouche, la fibre optique ayant une deuxième extrémité et une longueur telle que la deuxième extrémité puisse sortir de la bouche lorsque le capteur est dans la bouche. La deuxième extrémité est pourvue d'un simple diffuseur de lumière qui répand dans l'environnement du patient la lumière reçue de la fibre. Le récepteur, placé de manière à recevoir à distance une fraction de la lumière issue du diffuseur, détecte la modulation de la lumière qu'il reçoit, modulation qui transporte l'information d'image radiologique. Le récepteur est là encore de préférence pourvu d'un filtre optique centré sur la couleur émise par l'émetteur.

Le diffuseur est de préférence une simple boule de matière translucide placée à l'extrémité de la fibre optique, de sorte que la lumière est émise presque omnidirectionnellement.

Il y a de préférence une deuxième fibre optique, servant à la réception d'informations en provenance du système d'exploitation et à destination du capteur. Cette fibre a une première extrémité en regard d'un récepteur de lumière solidaire du capteur qui se trouve dans la bouche et une deuxième extrémité hors de la bouche, raccordée à un diffuseur de lumière qui peut être le même que celui de la première fibre ou un autre.

L'invention porte non seulement sur le système radiologique mais aussi sur le capteur radiologique intra-oral lui-même comprenant des moyens de conversion d'une image radiologique en signaux électroniques numériques et une source de lumière modulée par ces signaux électroniques, permettant de transmettre par voie optique des informations sur l'image radiologique recueillie par le capteur, à destination d'un récepteur de lumière situé hors de la bouche.

D'autres caractéristiques et avantages de l'invention apparaîtront à la lecture de la description détaillée qui suit et qui est faite en référence aux dessins annexés dans lesquels :
- la figure 1 représente un système de radiologie dentaire selon l'invention ;
- la figure 2 représente une vue du système radiologique dans une variante de l'invention ;
- la figure 3 représente une vue en coupe d'une réalisation pratique du capteur d'image ;
- la figure 4 représente une vue de dessus du capteur.

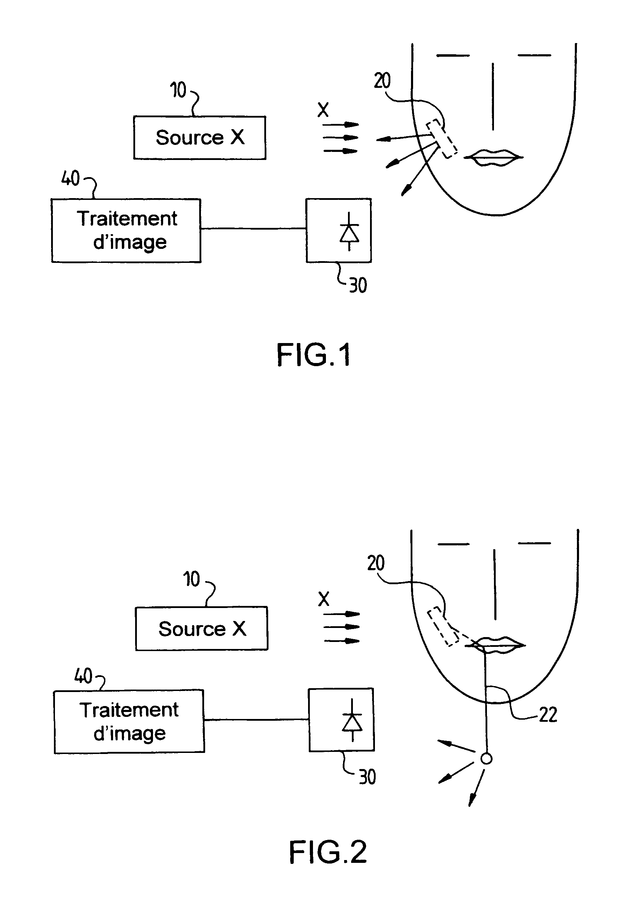

La figure 1 représente sous forme schématique le système de radiologie dentaire selon l'invention : il comporte une source de rayons X 10 capable d'émettre un flash de rayons X vers la partie de mâchoire à examiner, un capteur d'image radiologique 20 placé dans la bouche du patient et représenté en pointillés, un récepteur photosensible 30 placé à l'extérieur de la bouche du patient mais non relié électriquement au capteur, et un système d'analyse d'image 40 relié au récepteur et capable de recevoir des signaux lumineux numériques détectés par le récepteur, de les démoduler pour en extraire une information d'image qu'ils transportent, et de transformer les signaux démodulés en une image électronique. Cette image est destinée à être stockée dans une mémoire du système ou affichée sur un écran de visualisation non représenté.

Le capteur d'image radiologique comporte classiquement une couche scintillatrice, sensible aux rayons X et convertissant l'image X reçue en une image lumineuse, et une matrice de détection d'image lumineuse placée derrière la couche scintillatrice. La matrice fournit des signaux électroniques numériques (ou analogiques mais convertis ensuite en numérique) représentant l'image radiologique captée au moment du flash de rayons X.

De manière non classique, le capteur d'image est pourvu en outre d'une source d'émission lumineuse (de préférence une diode électroluminescente) et de moyens de modulation de cette source lumineuse. Les moyens de modulation reçoivent les signaux électroniques numériques issus de la matrice et représentant l'image radiologique à transmettre et modulent la lumière émise par la source en fonction de ces signaux numériques.

La source de lumière, dans le cas de la figure 1, émet à l'intérieur de la bouche ; on choisit la longueur d'onde de la source dans le rouge ou le proche infrarouge (longueurs d'onde pour laquelle la chair du patient (joues notamment) présente une certaine transparence. Il sort de la peau du patient, et/ou de la bouche si elle est ouverte, une fraction de puissance lumineuse modulée. Cette puissance lumineuse est émise de manière non directionnelle.

Le récepteur photosensible recueille une partie de cette fraction de puissance, la convertit en signaux électroniques et la transmet aux moyens d'analyse d'image. Le récepteur est de préférence pourvu d'un filtre de sélection de longueur d'onde centré sur la longueur d'onde principale émise par la source de lumière, afin que d'autres sources de lumière présentes dans l'environnement perturbent le moins possible la réception. La bande passante du filtre peut être de l'ordre de 30 nanomètres. Le récepteur peut être placé assez près du patient, par exemple à quelques dizaines de centimètres ou moins. En effet, il peut être porté par une partie du système qui porte également la source à rayons X. Or on sait que la source à rayons X est placée en général sur un bras articulé qui lui permet de s'approcher quelques centimètre de la joue. Le récepteur peut être porté par le même bras articulé.

Dans une variante préférée, représentée à la figure 2, on s'affranchit des difficultés de transmission optique à travers les joues du patient, et on prévoit une fibre optique 22 dont une première extrémité est fixée en regard de la source de lumière (à l'intérieur de la bouche du patient) et dont une autre extrémité sort à l'extérieur de la bouche. Cette autre extrémité est diffusante de manière que la lumière recueillie par la fibre depuis la source soit émise à l'extérieur de manière non directionnelle ou en tout cas avec une faible directivité. Une boule translucide peut être collée à cette extrémité pour jouer ce rôle de diffuseur de lumière. La boule peut être en alumine ou en matière plastique telle que du nylon blanc. Elle peut-être une boule d'environ 1 mm de diamètre protégée par une coque en matière plastique surmoulée, transparente à la longueur d'onde considérée.

Une fraction de la lumière émise est recueillie, à distance, par le récepteur et est transmise au système d'analyse d'image 40.

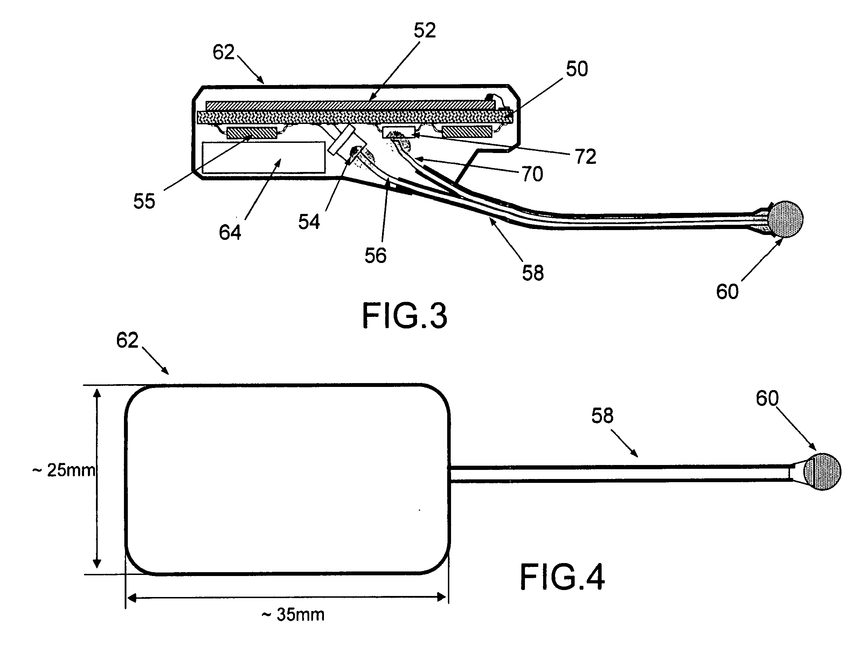

Sur la figure 3, on voit le détail d'un capteur intra oral selon l'invention, dans une réalisation avec fibres optiques sortant de la bouche du patient.

Le capteur comporte un circuit imprimé 50 portant sur une face une puce de circuit-intégré 52 sur une face de laquelle est formé le capteur d'image radiologique proprement dit, à savoir une matrice de détection d'image lumineuse recouverte d'un scintillateur réagissant aux rayons X. La matrice établit des signaux électroniques représentant les niveaux lumineux des points d'image ; la puce 52, de quelques centimètres de côté, comporte les circuits de conversion analogique-numérique permettant de convertir l'image électronique en signaux numériques représentant chaque point d'image.

Ces signaux sont appliqués à une diode électroluminescente (LED) 54 ou une diode laser, montée sur le circuit imprimé 50, de préférence sur l'autre face de ce dernier. La diode émet des impulsions lumineuses selon les signaux numériques qu'elle reçoit de la puce 52. La lumière émise est de préférence une lumière infrarouge dans une longueur d'onde non dangereuse pour les yeux et de préférence assez fortement monochromatique. Le circuit imprimé 52 peut comporter d'autres éléments de circuiterie tels que le composant 55 représenté sur la figure 3.

Une fibre optique 56, contenue dans une gaine souple de matière plastique 58 d'une dizaine de centimètres de long, possède une extrémité collée (par une colle optique transparente) devant la face d'émission de la diode 54. Cette fibre possède une autre extrémité collée par une colle optique transparente à un diffuseur de lumière 60. Le diffuseur 60 est une boule de matière translucide (matière plastique telle que du nylon blanc ou alumine). Sa forme sensiblement sphérique permet que la lumière issue de la fibre optique soit répartie dans un large angle solide afin qu'il n'y ait pas besoin de rechercher une orientation spécifique de la fibre optique pour que le récepteur 30 (figure 2) reçoive la lumière émise. La fibre optique gainée, n'étant attachée à rien à sa deuxième extrémité, ne risque pas d'être arrachée par un mouvement intempestif. Le diamètre de la fibre gainée est d'environ 1 mm de diamètre.

L'ensemble du circuit imprimé 50 et de ses composants, avec la puce de circuit intégré 52, la diode 54, et la première extrémité de la fibre optique gainée, est logé dans un boîtier étanche 62 d'où sort la fibre optique. Le boîtier contient de préférence aussi une pile ou une batterie 64 permettant l'alimentation électrique autonome du circuit imprimé et des composants qu'il porte. On peut aussi prévoir que le boîtier est en matière plastique transparente et que la fibre optique gainée est collée sur le boîtier, en regard de la diode laser 54, au lieu de pénétrer dans le boîtier.

Dans un exemple de réalisation, les dimensions du boîtier du capteur sont les suivantes : L= environ 35 mm, I= environ 25mm, H=environ 10mm.

Optionnellement, on peut prévoir que le système radiologique comporte aussi des moyens de communication optique dans le sens inverse, à savoir depuis le système vers le capteur placé dans la bouche. Cette option est prévue dans le capteur de la figure 3 sous forme d'une deuxième fibre optique 70 et d'une photodiode 72. La photodiode est montée sur le circuit imprimé 50. La deuxième fibre optique est de préférence montée dans la même gaine que la première, elle a une première extrémité collée avec une colle optique sur la même boule de diffusion 60 de manière à recueillir de la lumière arrivant un peu dans n'importe quelle direction. La photodiode peut recevoir et faire traiter (en particulier par la puce de circuit-intégré 52) des informations ou instructions en provenance du système. Un émetteur de lumière associé au système peut émettre des impulsions lumineuses convoyant ces informations ou instructions, la longueur d'onde de la lumière émise à cet effet étant de préférence différente de la longueur d'onde émise par la diode laser 54 afin qu'il n'y ait pas d'interférence nuisible entre les impulsions lumineuses aller et les impulsions lumineuses retour.

A titre d'exemple les impulsions de retour (du système vers le capteur) peuvent servir à envoyer une information de déclenchement d'un flash X, ou bien à demander au capteur d'envoyer ou renvoyer une image ou une partie d'image, ou encore à paramétrer certaines fonctions du capteur (temps d'exposition, etc.). Le débit d'informations dans le sens de retour peut être beaucoup plus faible que dans le sens aller puisqu'il n'y a pas d'image à transmettre.

On notera qu'un système bidirectionnel peut être prévu même avec une seule fibre optique, pourvu qu'il y ait à la sortie de la fibre des moyens de séparation pour transmettre les informations reçues à un récepteur de lumière placé sur le capteur en les séparant des informations émises ; la séparation peut se faire par exemple à l'aide d'un miroir dichroïque ; elle peut se faire aussi avec un miroir classique, avec une séparation temporelle en utilisant un protocole qui éteint périodiquement la source interne de lumière pendant des instants réservés à la réception d'informations en provenance de l'extérieur de la bouche du patient. La diode laser peut elle-même servir de photodiode si elle n'est pas elle-même en cours d'émission.

## Revendications

1. Système de radiologie dentaire par voie intra orale comportant un capteur d'image radiologique apte à être inséré dans la bouche du patient et comportant d'une part une matrice de détection d'image (52) fournissant des signaux électroniques représentant une image radiologique et **caracterisé par le fait qu'**il comprenne d'autre part une source de lumière (54) recevant les signaux issus de la matrice et apte à émettre des impulsions lumineuses binaires correspondant à une image numérique à transmettre par voie optique, et un récepteur de lumière (30) placé à distance du patient, apte à détecter une modulation de lumière provoquée par la source de lumière placée dans la bouche, et apte à transmettre un signal correspondant à cette modulation à un dispositif de traitement d'image (40).

2. Système selon la revendication 1, **caractérisé en ce que** la source de lumière (54) est placée sur le capteur et émet de la lumière à l'intérieur de la bouche du patient lorsque le capteur est placé à l'intérieur de la bouche, à une longueur d'onde et avec une puissance telle qu'une fraction de la lumière émise passe à travers les joues.

3. Système selon la revendication 2, **caractérisé en ce que** la source de lumière (54) est une diode laser ou une diode électroluminescente émettant une lumière sensiblement monochromatique et le récepteur est pourvu d'un filtre optique à bande passante étroite centrée autour de la longueur d'onde de la lumière monochromatique.

4. Système selon la revendication 3, **caractérisé en ce que** la longueur d'onde utilisée est dans la bande de 780 à 880 nanomètres (rouge ou proche-infrarouge), bande dans laquelle la peau des joues laisse passer une fraction notable de la lumière émise.

5. Système selon la revendication 1, **caractérisé en ce que** la source de lumière (54) est raccordée à une première extrémité d'une fibre optique (56), à l'intérieur de la bouche, la fibre optique ayant une deuxième extrémité et une longueur telle que la deuxième extrémité puisse sortir de la bouche lorsque le capteur est dans la bouche.

6. Système selon la revendication 1, **caractérisé en ce que** la deuxième extrémité de la fibre est pourvue d'un simple diffuseur de lumière (60) qui répand dans l'environnement du patient la lumière reçue de la fibre.

7. Système selon la revendication 6, **caractérisé en ce que** le diffuseur est une boule de matière translucide placée à l'extrémité de la fibre optique.

8. Système selon l'une des revendications 5 à 7, **caractérisé en ce que** le capteur comporte des moyens pour recevoir des informations ou instructions en provenance du système d'exploitation et à destination du capteur, ces informations étant reçues sous forme optique par la fibre optique et transmises à un récepteur de lumière, des moyens de séparation étant prévus pour séparer, à la première extrémité de la fibre optique, les informations émises des informations reçues et pour transmettre les informations reçues au récepteur de lumière.

9. Système selon l'une des revendications 6 et 7, **caractérisé en ce qu'**il comporte une deuxième fibre optique (70), servant à la réception d'informations ou d'instructions en provenance du système d'exploitation et à destination du capteur, et **en ce que** le capteur comporte un récepteur de lumière (72) en regard d'une extrémité de la deuxième fibre optique, la fibre optique ayant une longueur telle qu'une autre extrémité puisse sortir de la bouche lorsque le capteur est dans la bouche.

10. Système selon la revendication 9, **caractérisé en ce que** l'autre extrémité de la deuxième fibre optique est raccordée à un diffuseur de lumière.

## Claims

1. An intraoral dental radiology system comprising a radiological image sensor adapted to be inserted into the patient's mouth and comprising on the one hand an image detection matrix (52) delivering electrical signals representing a radiological image and on the other hand a light source (54) receiving the signals coming from the matrix and adapted to emit binary light pulses corresponding to a digital image to be transmitted optically, and a light receiver (30) placed at a distance from the patient, adapted to detect a light modulation caused by the light source placed in the mouth and adapted to transmit a signal corresponding to this modulation to an image processing device (40).

2. The system as claimed in claim 1, **characterized in that** the light source (54) is placed on the sensor and emits light inside the patient's mouth when the sensor is placed inside the mouth, at a wavelength and with a power such that a fraction of the emitted light can pass through the cheeks.

3. The system as claimed in claim 2, **characterized in that** the light source (54) is a laser diode or a light-emitting diode emitting substantially monochromatic light and the receiver is provided with an optical filter of narrow passband centered around the wavelength of the monochromatic light.

4. The system as claimed in claim 3, **characterized in that** the wavelength used is in the band from 780 to 880 nanometers (red or near infrared), in which band the skin of the cheeks allows a significant fraction of the emitted light to pass through.

5. The system as claimed in claim 1, **characterized in that** the light source (54) is connected to a first end of an optical fiber (56) inside the mouth, the optical fiber having a second end and a length such that the second end can emerge from the mouth when the sensor is in the mouth.

6. The system as claimed in claim 1, **characterized in that** the second end of the fiber is provided with a simple light diffuser (60) which spreads the light received from the fiber into the environment of the patient.

7. The system as claimed in claim 6, **characterized in that** the diffuser is a ball of translucent material placed at the end of the optical fiber.

8. The system as claimed in one of claims 5 to 7, **characterized in that** the sensor comprises means for receiving information or instructions coming from the operating system and intended for the sensor, this information being received in optical form by the optical fiber and transmitted to a light receiver, splitting means being provided at the first end of the optical fiber in order to separate the emitted information from the received information and to transmit the received information to the light receiver.

9. The system as claimed in one of claims 6 and 7, **characterized in that** it comprises a second optical fiber (70) used to receive information or instructions coming from the operating system and intended for the sensor, and **in that** the sensor comprises a light receiver (72) facing one end of the second optical fiber, the optical fiber having a length such that the other end can emerge from the mouth when the sensor is in the mouth.

10. The system as claimed in claim 9, **characterized in that** the second end of the second optical fiber is connected to a light diffuser.

## Patentansprüche

1. Dentalradiologisches System über den intraoralen Weg, das einen bildgebenden radiologischen Sensor aufweist, der imstande ist, in den Mund des Patienten eingeführt zu werden und einerseits eine Bilderfassungsmatrix (52) aufweist, die elektronische Signale liefert, die ein radiologisches Bild darstellen und **dadurch gekennzeichnet ist, dass** es andererseits eine Lichtquelle (54) umfasst, die die von der Matrix ausgehenden Signale empfängt und imstande ist, binäre Lichtimpulse zu senden, die einem auf optischem Weg zu übertragenden Digitalbild entsprechen, und einen Lichtempfänger (30), der entfernt vom Patienten platziert ist, imstande, eine Lichtmodulation zu erfassen, die von der im Mund platzierten Lichtquelle hervorgerufen wird, und imstande, ein Signal, das dieser Modulation entspricht, an eine Bildverarbeitungsvorrichtung (40) zu übertragen.

2. System nach Anspruch 1, **dadurch gekennzeichnet, dass** die Lichtquelle (54) auf dem Sensor platziert ist und Licht im Mund des Patienten aussendet, wenn der Sensor im Mund platziert ist, mit einer Wellenlänge und einer Leistung, die derart ist, dass ein Teil des ausgesendeten Lichts die Wangen durchquert.

3. System nach Anspruch 2, **dadurch gekennzeichnet, dass** die Lichtquelle (54) eine Laserdiode oder eine Leuchtdiode ist, die ein etwa monochromatisches Licht aussendet und der Empfänger mit einem optischen Filter mit einer engen Bandbreite ausgestattet ist, die um die Wellenlänge des monochromatischen Lichts zentriert ist.

4. System nach Anspruch 3, **dadurch gekennzeichnet, dass** die verwendete Wellenlänge im Bandbereich von 780 bis 880 Nanometer ist (rot oder nahes infrarot), wobei die Haut der Wangen in diesem Bandbereich einen erheblichen Teil des ausgesendeten Lichts durchgehen lässt.

5. System nach Anspruch 1, **dadurch gekennzeichnet, dass** die Lichtquelle (54) im Mund mit einem ersten Ende einer optischen Faser (56) verbunden ist, wobei die optische Faser ein zweites Ende hat und eine Länge, die derart ist, dass das zweite Ende aus dem Mund austreten kann, wenn der Sensor im Mund ist.

6. System nach Anspruch 1, **dadurch gekennzeichnet, dass** das zweite Faserende mit einem einfachen Lichtverteiler (60) ausgestattet ist, der das von der Faser empfangene Licht in einem Umfeld des Patienten streut.

7. System nach Anspruch 6, **dadurch gekennzeichnet, dass** der Verteiler eine Kugel aus durchscheinendem Material ist, die am Ende der optischen Faser platziert ist.

8. System nach einem der Ansprüche 5 bis 7, **dadurch gekennzeichnet, dass** der Sensor Mittel aufweist, um Informationen oder Befehle vom Betriebssystem und für den Sensor zu empfangen, wobei diese Informationen in optischer Form von der optischen Faser empfangen werden und an einen Lichtempfänger übermittelt werden, wobei Trennmittel vorgesehen sind, um am ersten Ende der optischen Faser die gesendeten Informationen von den empfangenen Informationen zu trennen und um die empfangenen Informationen an den Lichtempfänger zu übermitteln.

9. System nach einem der Ansprüche 6 und 7, **dadurch gekennzeichnet, dass** es eine zweite optische Faser (70) aufweist, die für den Empfang von Informationen oder Befehlen vom Betriebssystem und für den Sensor dient, und dass der Sensor einen Lichtempfänger (72) gegenüber einem Ende der zweiten optischen Faser aufweist, wobei die optische Faser eine Wellenlänge hat, die derart ist, dass ein anderes Ende aus dem Mund austreten kann, wenn der Sensor im Mund ist.

10. System nach Anspruch 9, **dadurch gekennzeichnet, dass** das andere Ende der zweiten optischen Faser mit einem Lichtverteiler verbunden ist.
